# FASCICULE DE BREVET EUROPEEN

(11) **EP 2 379 173 B1**
(45) Date de publication et mention de la délivrance du brevet: **15.03.2017**
(21) Numéro de dépôt: 10706696.1
(22) Date de dépôt: 07.01.2010
(51) Int. Cl.: A61P 11/00, A61K 31/785, A61K 45/06

(54) **COMPOSITION ANTI-RONFLEMENT CONTENANT UN POLYMÈRE THERMOGÉLIFIANT**
ANTI-SCHNARCH-ZUSAMMENSETZUNG MIT EINEM THERMOGELIERENDEN POLYMER
ANTI-SNORING COMPOSITION CONTAINING A THERMOGELLING POLYMER

(30) Priorité: 07.01.2009 FR 0950057
(43) Date de publication de la demande: 26.10.2011
(73) Titulaire: Polymerexpert S.A., 33600 Pessac (FR)
(72) Inventeur: DOLATKHANI, Marc, F-33610 Cestas (FR); PAGNOUX, Anne, F-33114 Le Barp (FR)
(74) Mandataire: Santarelli
(86) Numéro de dépôt international: PCT/FR2010/050021
(87) Numéro de publication internationale: WO 2010/079305

(56) Documents cités:
- WO-A-92/09307
- WO-A-03/106536
- WO-A-2006/122183
- WO-A-2007/138224
- WO-A1-98/29487
- CHOW YUNG-CHIONG ET AL: "Prophylactic intravesical instillation of epinephrine prevents cyclophosphamide-induced hemorrhagic cystitis in rats" EXPERIMENTAL BIOLOGY AND MEDICINE (MAYWOOD), vol. 232, no. 4, avril 2007 (2007-04), pages 565-570, XP002537238 ISSN: 1535-3702
- TRONG LIEM CHAU PHAM ET AL: "Mechanisms of micellization and rheology of PEO-PPO-PEO triblock copolymers with various architectures." JOURNAL OF COLLOID AND INTERFACE SCIENCE 15 DEC 2008, vol. 328, no. 2, 15 décembre 2008 (2008-12-15), pages 278-287, XP002537239 ISSN: 1095-7103

## Description

L'invention concerne la mise au point d'une formulation thermogélifiante pour la prévention et/ou le traitement du ronflement et administrable par vaporisation par les voies nasale ou buccale, selon l'étendu du jeu de revendications.

Le ronflement est lié à des problèmes respiratoires durant le sommeil. Le bruit produit, qui peut atteindre 100 décibels, est le résultat d'une vibration des tissus naso-pharyngés. L'air qui pénètre par le nez et la bouche doit traverser un passage étroit entre la langue, le voile du palais, la luette et la paroi du pharynx. Lors du sommeil, les muscles sont relâchés et ont tendance à s'affaisser, ce qui réduit encore le passage. Ces structures molles s'accolent l'une contre l'autre et empêchent l'air de passer librement. Les vibrations des tissus se traduisent par le ronflement. Le ronflement existe également en cas de rétrécissement des voies nasales.

Les ronflements sont particulièrement fréquents chez les adultes de plus de 40 ans et sont favorisés par la fatigue, le surpoids, la prise d'alcool ou de certains médicaments. On estime que 60% des hommes et 40% des femmes de plus de 40 ans ronflent épisodiquement et qu'environ 25% d'hommes et 15% de femmes souffrent d'un ronflement pathologique important appelé ronchopathie.

Les traitements préconisés peuvent être de différente nature :
- dormir sur le côté ou sur le ventre : l'espace pour le passage de l'air est ainsi augmenté,
- se reposer et éviter de boire de l'alcool ou prendre certains médicaments
- maigrir : la réduction du volume de la base de la langue et des tissus pharyngés améliore le passage de l'air.
- utiliser des systèmes favorisant le passage de l'air (écarteur narinaire, canule oropharyngée, orthèse d'avancée mandibulaire, etc.)
- la chirurgie : la pharyngo-plastie consistant à sectionner le bord du voile du palais et la luette ;
- l'utilisation de vaporisateurs (sprays) buccaux et nasaux dont l'action consiste à lubrifier pendant le sommeil les tissus mous, évitant ainsi les vibrations à l'origine du ronflement.

Les produits proposés en vaporisateurs (sprays) développés à ce jour et disponibles sur le marché présentent une efficacité limitée sur le ronflement en raison de leur composition. En effet, ce type de produit doit présenter un pouvoir lubrifiant élevé et une adhésivité importante sur les tissus afin d'assurer son maintien pendant la durée du sommeil.

Deux types de composition ont principalement été décrits dans la littérature, à savoir :
a) les préparations à base d'huile
   L'utilisation d'huiles alimentaires permet de lubrifier les muqueuses. Cependant la durée d'action de ce type de produit est très faible en raison de la faible affinité de l'huile (hydrophobe) avec les muqueuses hydrophiles. En effet, les gouttelettes d'huile sont très rapidement entraînées vers l'estomac, aidées par le phénomène de déglutition. Un autre inconvénient pour l'utilisation de ce type de préparation est le risque de pneumopathie huileuse lié au passage de gouttelettes d'huile dans les poumons.
b) les préparations à base de polysaccharides ou de dérivés acryliques.

L'utilisation de polysaccharide dans la composition de produits anti-ronflement comme agent muco-adhésif et/ou d'agent lubrifiant a été décrite, par exemple, dans les demandes de brevet FR 2 859 105, WO 2006/042926 et WO 2007/138224. Parmi ces polysaccharides figure l'acide hyaluronique utilisé depuis des années dans le domaine ophtalmique comme agent lubrifiant et dont les propriétés muco-adhésives sont longuement décrites dans la littérature. D'autres polysaccharides, comme les carraghénanes, sont utilisés uniquement pour leurs propriétés muco-adhésives et sont associés à des agents lubrifiants.

Tout comme les polysaccharides, les dérivés acryliques cités sont hydrosolubles, et sont rapidement lessivés par le flux de salive, ce qui constitue l'inconvénient majeur de cette approche. Ce point est d'autant plus vrai que ces polymères sont utilisés dans de faibles proportions parce qu'ils viscosifient les formulations de manière importante (même à faible taux), rendant difficile l'aptitude des produits à être vaporisés. L'inconvénient majeur de ces préparations concerne leur aptitude à être rapidement lessivées par le flux de salive.

L'invention concerne donc une composition, apte à être vaporisée, destinée à prévenir et/ou traiter efficacement et durablement le ronflement, comprenant au moins un polymère thermogélifiant présentant à la fois des propriétés muco-adhésives et lubrifiantes. L'approche consiste à vaporiser un produit liquide à température ambiante par voie nasale ou buccale qui se gélifie au contact des muqueuses assurant le maintien du produit durant la période de sommeil.

L'invention concerne également l'utilisation d'au moins un polymère thermogélifiant dans une composition pour la prévention et/ou le traitement du ronflement.

Plus particulièrement, l'invention a pour objet une composition comprenant au moins un polymère thermogélifiant en solution aqueuse, ledit polymère thermogélifiant comprenant d'une part des unités hydrosolubles et d'autre part des unités présentant dans l'eau une température inférieure critique de démixtion et étant présent à une concentration massique inférieure ou égale à 10% dans ladite solution aqueuse, pour la prévention et/ou le traitement du ronflement.

On entend par « polymères thermogélifiants » des polymères qui, à faible concentration dans l'eau, se présentent sous forme liquide (faible viscosité) à température ambiante et qui gélifient à la température corporelle : le phénomène de thermogélification étant parfaitement réversible.

Avantageusement, la solution aqueuse de polymère thermogélifiant utilisable aux fins de l'invention présente une viscosité inférieure à 0,1 Pas sous 10s⁻¹ de cisaillement à température ambiante, et une viscosité supérieure à 0,1 Pa.s, de préférence supérieure à 0,3 Pa.s sous 10s⁻¹ de cisaillement au contact des muqueuses, à la température corporelle.

Par « température ambiante », on entend une température de l'ordre de 18 à 25°C.

La concentration massique en polymère thermogélifiant en solution aqueuse est inférieure ou égale à 10%, de préférence comprise entre 2 et 10%.

On notera que les valeurs de viscosité de la solution aqueuse de polymère thermogélifiant sont données ci-dessus pour un cisaillement de 10^{s-1} (cisaillement appliqué lors de la vaporisation). Ledit polymère étant rhéofluidifiant, sa viscosité diminue avec le cisaillement. Cependant, le cisaillement dans les voies nasales ou buccales étant pratiquement nul, la viscosité effective au contact des muqueuses nasales ou buccales est beaucoup plus élevée, ce qui permet à ladite solution aqueuse de polymère thermogélifiant de se présenter sous forme de gel.

Les polymères thermogélifiants utilisables aux fins de l'invention comprennent, d'une part, des unités hydrosolubles et, d'autre part, des unités présentant dans l'eau une température inférieure critique de démixtion (appelée en abrégé « LCST » pour « lower critical solution temperature »). En dessous de la LCST, le polymère est parfaitement soluble dans l'eau, alors qu'au dessus de cette température les portions à LCST s'associent et perdent leur solubilité dans l'eau, formant ainsi des noeuds de réticulation entre les chaînes polymères. Le polymère s'apparente alors à un réseau tridimensionnel, ce qui conduit à l'obtention d'un gel. Cette association de chaînes à LCST au sein des micro-domaines hydrophobes au-delà de la température de démixtion étant de nature physique, le phénomène de gélification est alors parfaitement réversible.

Cette propriété de gélification avec la température n'est possible
que si la concentration en polymère est supérieure à la concentration micellaire critique pour permettre des interactions entre les unités à LCST portées par des chaînes polymères différentes.

Les polymères utilisés dans l'invention peuvent être aussi bien des polymères à blocs ou des polymères greffés qui comprennent, d'une part, des unités hydrosolubles et d'autre part des unités à LCST.

Ces unités hydrosolubles peuvent être des polymères d'origine naturelle ou d'origine synthétique obtenus par polymérisation en chaîne ou par polycondensation.

Les unités à LCST seront préférentiellement choisies parmi :
- les polyéthers tels que le poly(oxyde de propylène) POP et ses copolymères statistiques et à blocs avec le poly(oxyde d'éthylène)
- les dérivés N-substitués de l'acrylamide comme le poly(N-isopropylacrylamide) ou le poly (N-éthylacrylamide)
- le polyvinylcaprolactame et les copolymères de vinylcaprolactame.

Les polymères thermogélifiants susceptibles de convenir peuvent être, par exemple, choisis parmi ceux décrits dans les demandes de brevets et brevets suivants : FR2694939, FR2788008, FR2820976, FR2856923, GB2408510, EP0629649, EP1307501, EP1407791, WO97/00275, WO98/06438, WO98/29487, WO98/48768, WO98/50005, WO00/00222, WO00/07603, WO00/35961, WO00/38851, WO01/41735, WO02/032560, WO02/076392, WO03/008462, WO03/106536, WO04/006872, US2003/0099709, US6645476, US6689855, US6689856, US6870012 et US6878754.

Les polymères thermogélifiants adaptés aux compositions servant à lutter contre le ronflement selon l'invention sont les polyuréthanes comportant des groupements poly(oxyde d'éthylène-b-oxyde de propylène-b-oxyde d'éthylène) (POE-b-POP-b-POE) tels que ceux décrits dans les brevets et les demandes de brevets FR2840907, EP692506, EP1407791, WO 03 106536, US 7339013.

Ces polyuréthanes sont obtenus par polycondensation de diisocyanates et de diols triblocs POE-b-POP-b-POE thermosensibles en milieu non anhydre et peuvent comprendre des groupements urée et/ou allophanates. Ces polymères connus sous le nom d'ExpertGel® et commercialisés par la société PolymerExpert présentent de nombreux avantages par rapport aux polymères de type Poloxamer® et aux viscosifiants classiques en phase aqueuse, en particulier:
- une faible concentration de polymères est nécessaire pour une augmentation importante de la viscosité en fonction de la température,
- ils présentent des propriétés lubrifiantes importantes,
- ils sont muco-adhésifs,
- ils présentent le même comportement thermogélifiant pour dans une gamme de pH allant de 1 à 12,
- ils sont compatibles avec les sels, tensio-actifs et la plupart des excipients pharmaceutiques et cosmétiques,
- Ils présentent un comportement rhéofluidifiant rendant possible leur vaporisation à température élevée (dans une salle de bain surchauffée, en plein soleil etc....)
- ils ont une température de gélification ajustable de manière précise entre 10 et 60°C,
- ce sont des produits non toxiques, qui peuvent être utilisés sans restriction dans des applications au contact de la peau et des muqueuses.

Les solutions de polymères de type ExpertGel® sont donc particulièrement adaptées à l'application visée.

Le premier intérêt concerne le mode de délivrance par vaporisation. En effet, la composition contenant les polymères thermogélifiants est liquide dans son conditionnement à température ambiante et devient gel au contact des muqueuses à 37°C. La thermogélification permet de cibler de manière précise la zone à traiter évitant toute coulure ou migration du produit et rend le produit vaporisé non lessivable augmentant la durée de sa présence dans la zone de traitement.

Le second intérêt de l'utilisation de ces polymères est lié à leur propriétés muco-adhésives exceptionnelles, comme le montrent les travaux du Pr. Jean-Louis Grossiord et al. dans J. Drug Del. Sci. Tech., 16(1) 59-4 2006.

Les auteurs ont comparé l'adhésion de deux types de polymère ExpertGel®, à savoir EG 30 et EG 40, avec celle du CARBOPOL 974P, aussi bien sur des supports synthétiques que sur les muqueuses intestinales du rat. Le CARBOPOL 974 qui est un polyacide acrylique partiellement neutralisé est connu dans le milieu pharmaceutique comme un polymère de référence pour ses propriétés bioadhésives. Les résultats montrent que la force du détachement à 37°C dans leurs conditions opératoires est de 0,2 N pour le CARBOPOL 974P alors qu'elle est de 0,7 N pour l'EG 35 et de 0,3 pour l'EG 40. Ils concluent donc que les deux types de polymères EG, et plus particulièrement l'EG35, présentent des propriétés muco-adhésives meilleures que le CARBOPOL 974P à 37°C, ce qui fait de ces polymères thermogélifiants des produits intéressants pour le développement de formulations cosmétiques et pharmaceutiques au contact de la peau et des muqueuses.

Le pouvoir lubrifiant de ces polymères constitue évidemment un autre intérêt de leur utilisation pour l'application anti-ronflement. Les polymères thermogélifiants, en particulier les polymères thermogélifiants constitués essentiellement de copolymères à blocs POE-b-POP-b-POE présentent des propriétés tensioactives. Les propriétés lubrifiantes des « savons » sont connues depuis très longtemps. De nombreuses études montrent en particulier les propriétés de glisse apportées par l'hydratation des chaînons POE ; le phénomène décrit s'apparente à un processus d'«aquaplanning ». S'inspirant de ces travaux, la demanderesse a, par ailleurs, développé des revêtements à base d'ExpertGel® dans le domaine biomédical facilitant la pose d'implants ou de sondes. Les dispositifs revêtus des polymères de type ExpertGel® présentent des propriétés de glisse importante au contact des muqueuses et ne procurent pas de gêne au moment de la pose. Un exemple concerne les sondes urinaires qui revêtues par une couche d'ExpertGel® glissent parfaitement et peuvent être mises en place sans gêne pour le patient.

En résumé, les polymères thermogélifiants utilisables aux fins de l'invention permettent :
1) une facilité d'application par vaporisation,
2) le traitement d'une zone précise en raison de la gélification immédiate de la solution vaporisée,
3) une lubrification importante de la zone traitée,
4) une grande efficacité du traitement en raison de leurs propriétés muco-adhésives et une grande résistance du gel au phénomène de lessivage (gel peu soluble par le flux de salive).

Avantageusement, les compositions anti-ronflement selon l'invention sont donc aptes à être vaporisées, par voie nasale ou buccale, elles présentent des propriétés muco-adhésives leur permettant de résister au phénomène de lessivage par le flux de salive et elles présentent des propriétés de lubrification des muqueuses. Ces propriétés muco-adhésives et lubrifiantes sont supérieures à celles des produits disponibles sur le marché.

Un autre avantage lié à l'utilisation de polymères thermogélifiants est la possibilité de libérer de manière contrôlée un principe actif à partir de la composition les contenant. La solution de polymère contenant le principe actif se gélifie à 37°C. Le principe actif est enrobé par le gel formé et peut diffuser de manière contrôlée.

L'invention concerne donc également une composition pour la prévention et/ou le traitement du ronflement comprenant un polymère thermogélifiant, telle que décrite plus haut, contenant un principe actif qui est libéré de manière contrôlée.

On peut citer, par exemple, la libération d'un agent décongestionnant, d'un agent anti-inflammatoire ou d'un agent capable de traiter ou prévenir le ronflement, par exemple un agent vasoconstricteur, tel que l'extrait de Ruscus Aculealus, qui pourrait être encapsulé par le polymère thermogélifiant et libéré avec une pharmacocinétique contrôlée durant la période de sommeil.

Par « agent décongestionnant », on entend un produit qui favorise l'élimination de l'excès de sang », et, par « agent vasoconstricteur », on entend un produit qui diminue le calibre d'un vaisseau par contraction de ses fibres musculaires.

Comme mentionné dans les brevets et les demandes de brevets FR 2840907, EP1407791, EP692506, WO03106536 et US 7339013, l'élaboration des polyuréthanes thermogélifiants peut être ajustée à l'application recherchée. Ainsi, la température de gélification, la polarité de la macromolécule, la variation de la viscosité peuvent être ajustées en fonction des réactifs et des conditions de synthèse permettant d'enrober de manière efficace le principe actif et le libérer de manière contrôlée.

Un certain nombre de paramètres peuvent également être ajustés par la formulation de ces produits avec d'autres additifs.

Ainsi, les compositions anti-ronflement selon l'invention peuvent comprendre, de manière optionnelle, un ou plusieurs additifs choisis parmi :
- des électrolytes, tels que par exemple, le chlorure de sodium, le chlorure de magnésium, le fluorure de sodium etc. La présence de sels dans le milieu favorise la démixtion et abaisse la température de gélification ; Lesdits électrolytes peuvent être présents dans la composition, par exemple, jusqu'à 15% en poids ;
- des agents lubrifiants, tel que, par exemple, le glycérol. Le glycérol a également pour effet d'abaisser la température de gélification et son addition peut augmenter les propriétés de lubrification de la composition anti-ronflement. Lesdits agents lubrifiants peuvent être présents dans la composition, par exemple, jusqu'à 30% en poids ;
- des alcools, tels que, par exemple, des alcools comme l'éthanol ou l'isopropanol, en tant que conservateurs de la solution.

Ledit alcool sera utilisé de préférence à une concentration inférieure à 10% dans le milieu de formulation, en particulier de l'ordre de 5%.
- des agents tensioactifs non ioniques, tels que, par exemple, les Tween® ou les Brij® ; lesdits agents lubrifiants peuvent être présents dans la composition, par exemple, jusqu'à 10% en poids.

L'addition d'un tensio-actif non ionique présente un double effet. On observe tout d'abord un abaissement de la température de gélification. De manière plus surprenante, on constate un effet de synergie entre le tensio-actif et le polymère thermogélifiant, conduisant à une augmentation encore plus importante de la viscosité en fonction de la température.
- des polysaccharides, tels que, par exemple, l'acide hyaluronique, les alginates, en particulier l'alginate de sodium, les xanthanes etc.

L'ajout d'une très faible quantité de polysaccharide, notamment inférieure à 5% en poids, permet d'ajuster la viscosité de la composition anti-ronflement à température ambiante. Le polysaccharide est alors utilisé comme viscosifiant classique de la formulation.
- des agents actifs, par exemple des agents ayant une action anti-inflammatoire ou bien vasoconstrictrice.

L'invention est illustrée par les exemples qui suivent. Sauf indication contraire, les pourcentages sont exprimés en poids.

### Exemple 1 :

La composition anti-ronflement suivante a été préparée :

| | | |
|---|---|---|
| - EG56 SEC (ExpertGel®) | 3g | 6% |
| - Ethanol | 2,5g | 5% |
| - Menthol | 0,05g | 0,1% |
| - eau minérale | 44,4g | qsp 100% |

Le polymère thermogélifiant EG56SEC (numéro CAS 93665-35-1) est un polyuréthane-urée à base de POE-POP-POE contenant environ 75% de POE, dont la désignation INCI (International Nomenclature of Cosmetic Ingredients) est BIS-Methoxy PEG-13 PEG-438/PPG-110 SMDI Copolymer.

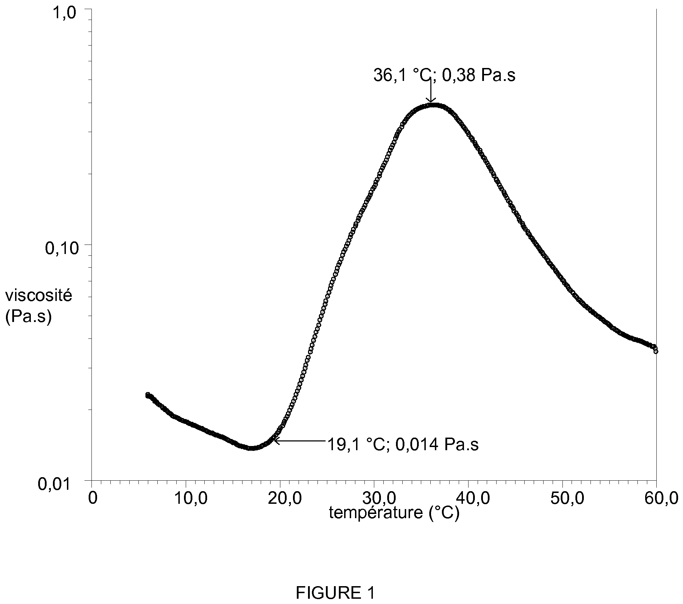

La courbe de viscosité en écoulement sous un cisaillement à 10s⁻¹ de la composition de l'exemple 1 en fonction de la température est représentée sur la figure 1.

### Exemple 2

La composition anti-ronflement suivante a été préparée :

| | | |
|---|---|---|
| - EG56 SEC (ExpertGel®) | 3g | 6% |
| - Ethanol | 2,5g | 5% |
| - Menthol | 0,05g | 0,1% |
| - Acide hyaluronique | 0,1g | 0,2% |
| - Eau minérale | 44,35g | qsp 100% |

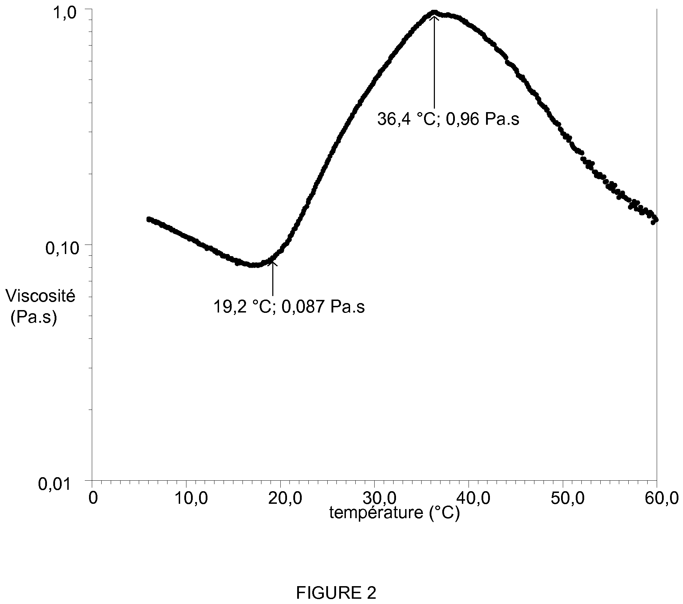

La courbe de viscosité en écoulement sous un cisaillement à 10s⁻¹de la composition de l'exemple 2 en fonction de la température est représentée sur la figure 2.

### Exemple 3

La composition anti-ronflement suivante a été préparée :

| | | |
|---|---|---|
| - EG56 SEC (ExpertGel®) | 3g | 6% |
| - Ethanol | 2,5g | 5% |
| - Menthol | 0,05g | 0,1% |
| - Acide hyaluronique | 0,1g | 0,2% |
| - Tween 20® | 0,05g | 0,1% |
| - Eau minérale | 44,4g | qsp 100% |

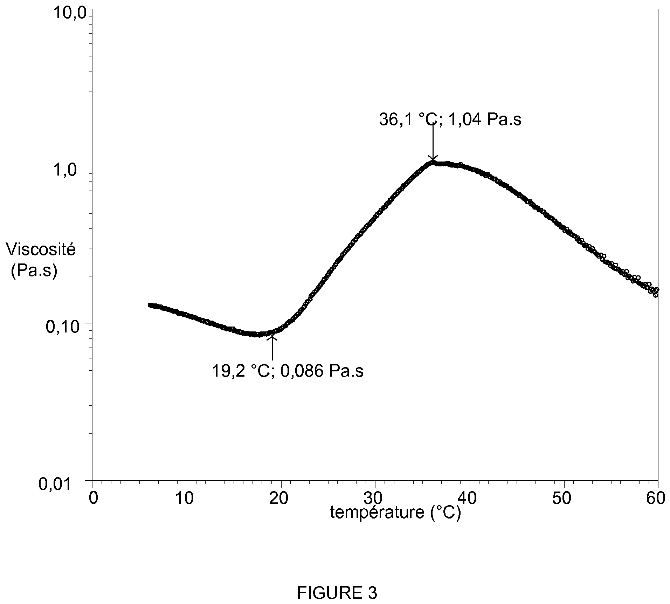

La courbe de viscosité en écoulement sous un cisaillement à 10s⁻¹ de la composition de l'exemple 3 en fonction de la température est représentée sur la figure 3.

### Exemple 4

La composition anti-ronflement suivante a été préparée :

| | | |
|---|---|---|
| - EG56 SEC (ExpertGel®) | 3g | 6% |
| - Ethanol | 2,5g | 5% |
| - Menthol | 0,05g | 0,1% |
| - Acide hyaluronique | 0,1g | 0,2% |
| - Tween 20® | 0,05g | 0,1% |
| - Chlorhexidine | 0,05g | 0,1% |
| - Eau minérale | 44,20g | qsp 100% |

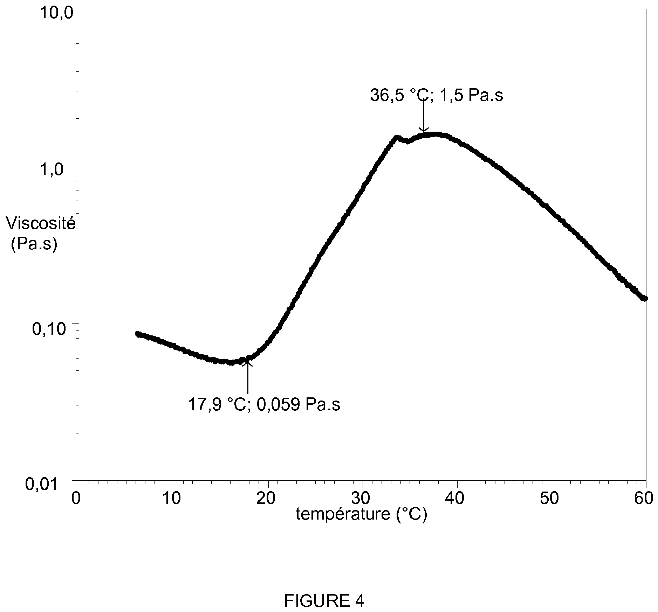

La courbe de viscosité en écoulement sous un cisaillement à 10s⁻¹ de la composition de l'exemple 4 en fonction de la température est représentée sur la figure 4.

### Exemple 5

La composition anti-ronflement suivante a été préparée :

| | | |
|---|---|---|
| - EG 230 (ExpertGel®) | 3,5g | 7% |
| - Ethanol | 2,5g | 5% |
| - Chlorure de sodium | 0,45g | 0,9% |
| - Menthol | 0,05g | 0,1% |
| - Tween 20® | 0,15g | 0,3% |
| - Eau minérale | 43,35g | qsp 100% |

Le polymère thermogélifiant EG230 (numéro CAS 93665-35-1) est un polyuréthane ramifié contenant des unités POE-b-POP-b-POE, dont la désignation INCI (International Nomenclature of Cosmetic Ingredients) est BIS-Methoxy PEG-13 PEG-502/PPG-57 SMDI Copolymer.

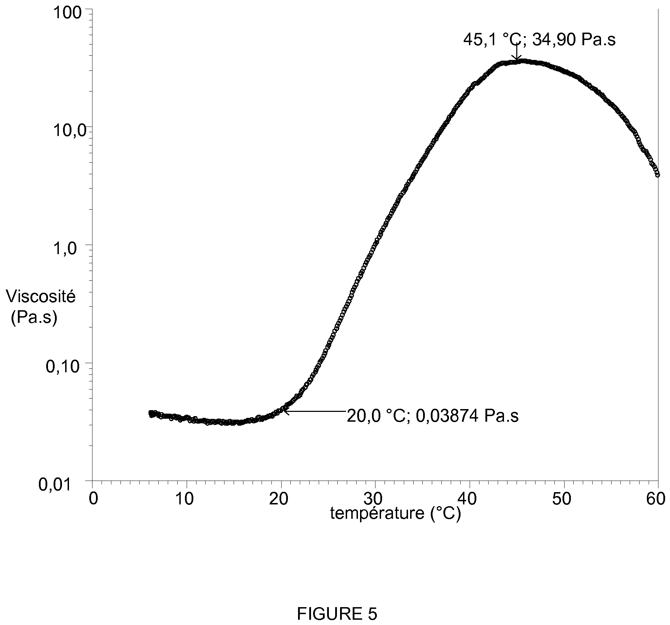

La courbe de viscosité en écoulement sous un cisaillement à 10s⁻¹ de la composition de l'exemple 5 en fonction de la température est représentée sur la figure 5.

### Exemple 6

La composition anti-ronflement suivante a été préparée :

| | | |
|---|---|---|
| - EG 230 (ExpertGel®) | 3,5g | 7% |
| - Ethanol | 2,5g | 5% |
| - Acide hyaluronique | 0,1g | 0,2% |
| - Chlorure de sodium | 0,45g | 0,9% |
| - Menthol | 0,05g | 0,1% |
| - Tween 20® | 0,15g | 0,3% |
| - Eau minérale | 43,25g | qsp 100% |

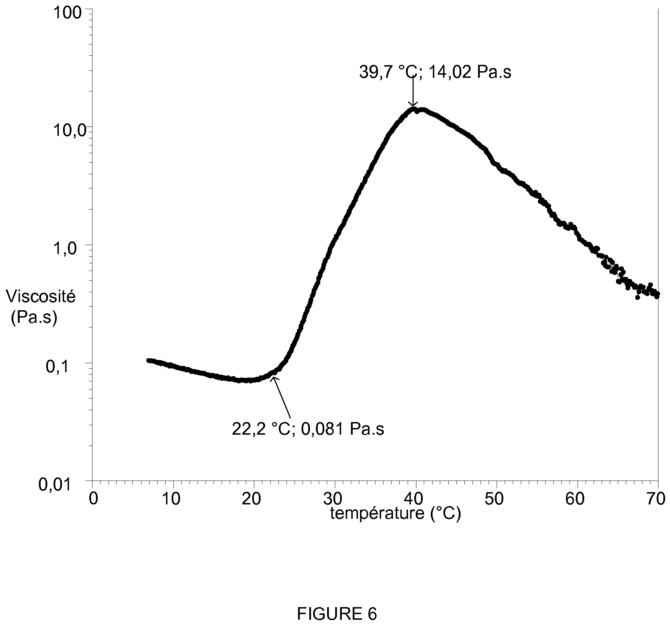

La courbe de viscosité en écoulement sous un cisaillement à 10s⁻¹ de la composition de l'exemple 6 en fonction de la température est représentée sur la figure 6.

### Exemple 7

La composition anti-ronflement suivante a été préparée :

| | | |
|---|---|---|
| - EG 230 (ExpertGel®) | 3,5g | 7% |
| - Ethanol | 2,5g | 5% |
| - Acide hyaluronique | 0,1g | 0,2% |
| - Chlorhexidine | 0,05g | 0,1% |
| - Chlorure de sodium | 0,45g | 0,9% |
| - Menthol | 0,05g | 0,1% |
| - Tween 20® | 0,15g | 0,3% |
| - Eau minérale | 43,2g | qsp 100% |

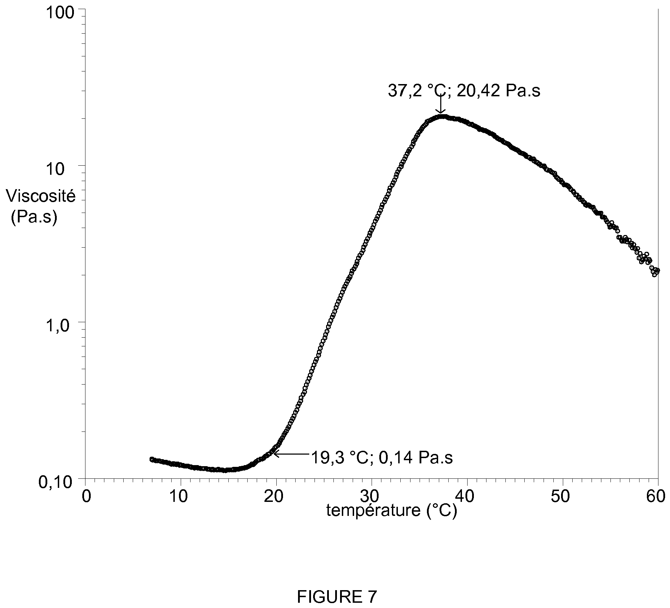

La courbe de viscosité en écoulement sous un cisaillement à 10s⁻¹ de la composition de l'exemple 7 en fonction de la température est représentée sur la figure 7.

### Exemple 8 : étude de la libération d'un principe actif dans une formulation contenant un polymère thermogélifiant

Un principe actif qui a été particulièrement étudié est l'acétylsalicylate de DL-Lysine (Aspégic®). La formulation rapportée dans le tableau 1 ci-dessous a été réalisée avec une solution aqueuse de polymère ExpertGel® n°EG56.

Le polymère thermogélifiant EG56 (numéro CAS 93665-35-1) est un polyuréthane ramifié contenant des unités POE-b-POP-b-POE, dont la désignation INCI (International Nomenclature of Cosmetic Ingrédients) est BIS-Methoxy PEG-13 PEG-438/PPG-110 SMDI Copolymer.

**Tableau 1**

| Polymère thermogélifiant ExpertGel® | [ExpertGel®] % p/p formulation | [Aspégic®] % p/p formulation | [Aspégic®] maximale dans l'eau à 37°C g/L |
|---|---|---|---|
| EG56 | 7,50 | 10 | 2,34 |

Cette solution a été ensuite versée goutte à goutte dans 20 ml d'eau placée à 37°C. Les gouttes gélifient immédiatement au contact de l'eau à 37°C et enrobent l'actif.

La cinétique de libération de l'Aspégic® à 37°C a été suivie par spectroscope UV-visible.

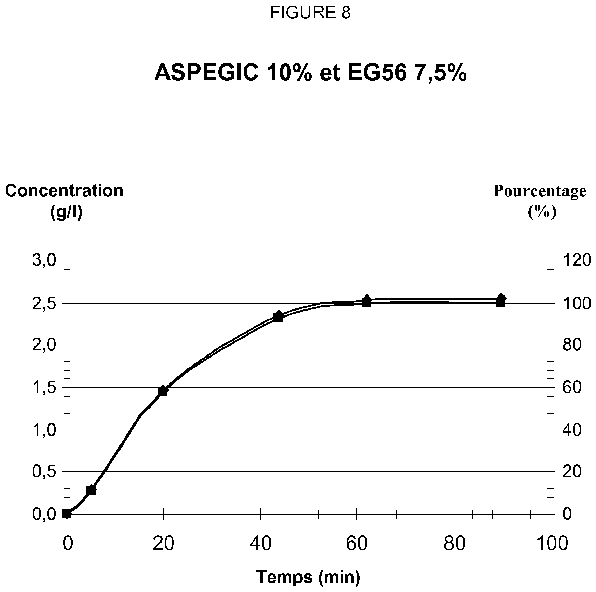

La courbe de la figure 8 représente la cinétique de libération à 37°C de l'Aspégic® encapsulé dans une solution d'EG56C à 7,5% dans de l'eau à 37°C, exprimée sur l'ordonnée de gauche (symbole -◆-) en concentration (g/l), et sur l'ordonnée de droite (symbole -■-) en pourcentage en poids (%), en fonction du temps (min).

Le tableau 2 rapporte la cinétique de libération de l'Aspégic® libre ou dans une solution aqueuse de polymère thermogélifiant.

**Tableau 2 :**

| Solution testée | Temps pour relarguer 50% d'Aspégic® | Temps pour relarguer 100% d'Aspégic® |
|---|---|---|
| 7,5%EG56 + 10% Aspégic® | 16 min | 62 min |
| Solution de 10% Aspégic® | 0 | 0 |

Les résultats montrent que l'utilisation de polymère thermogélifiant permet de contrôler la libération du principe actif.

## Revendications

1. Composition comprenant au moins un polymère thermogélifiant en solution aqueuse, ledit polymère thermogélifiant comprenant d'une part des unités hydrosolubles et d'autre part des unités présentant dans l'eau une température inférieure critique de démixtion et étant choisi parmi les polyuréthanes comportant des groupements poly(oxyde d'éthylène-b-oxyde de propylène-b-oxyde d'éthylène) (POE-b-POP-b-POE) et étant présent à une concentration massique inférieure ou égale à 10% dans ladite solution aqueuse, pour l'utilisation dans la prévention et/ou le traitement du ronflement.

2. Composition pour l'utilisation selon la revendication 1, **caractérisée en ce que** ledit polymère thermogélifiant présente, en solution aqueuse, une viscosité inférieure à 0,1 Pa.s sous 10s⁻¹ de cisaillement à température ambiante et une viscosité supérieure à 0,1 Pa.s, de préférence supérieure à 0,3 Pa.s, sous 10s⁻¹ de cisaillement au contact des muqueuses, à la température corporelle.

3. Composition pour l'utilisation selon l'une des revendications 1 ou 2, **caractérisée en ce que** ledit polymère thermogélifiant est présent à une concentration massique de 2 à 10% dans la solution aqueuse.

4. Composition pour l'utilisation selon l'une quelconque des revendications 1 à 3, **caractérisée en ce que** ledit polymère thermogélifiant est obtenu par polycondensation de diisocyanates et de diols triblocs POE-b-POP-b-POE et peuvent comprendre des groupements urée et/ou allophanates.

5. Composition pour l'utilisation selon l'une quelconque des revendications 1 à 4, **caractérisée en ce qu'**elle contient un principe actif qui est libéré de manière contrôlée.

6. Composition pour l'utilisation selon la revendication 5, **caractérisée en ce que** ledit principe actif est enrobé par le gel formé au contact des muqueuses et libéré de manière contrôlée.

7. Composition pour l'utilisation selon l'une des revendications 5 ou 6, **caractérisée en ce que** ledit principe actif est choisi parmi un agent décongestionnant, un agent anti-inflammatoire, un agent capable de traiter ou de prévenir le ronflement et un agent vasoconstricteur.

8. Composition pour l'utilisation selon l'une quelconque des revendications 1 à 7, **caractérisée en ce qu'**elle comprend au moins un additif choisi parmi
- des éléctrolytes,
- des agents lubrifiants,
- des alcools,
- des agents tensioactifs non ioniques, et
- des polysaccharides.

9. Composition pour l'utilisation selon l'une quelconque des revendications 1 à 8, **caractérisée en ce que** ce qu'elle est apte à être vaporisée, par voie nasale ou buccale.

10. Composition pour l'utilisation selon l'une quelconque des revendications 1 à 9, **caractérisée en ce que** ce qu'elle présente des propriétés muco-adhésives lui permettant de résister au phénomène de lessivage par le flux de salive.

11. Composition pour l'utilisation selon l'une quelconque des revendications 1 à 9, **caractérisée en ce que** ce qu'elle présente des propriétés de lubrification des muqueuses.

12. Utilisation d'au moins un polymère thermogélifiant tel que défini dans l'une quelconque des revendications 1 à 4 pour la préparation d'une composition pour la prévention et/ou le traitement du ronflement.

13. Utilisation selon la revendication 12, **caractérisé en ce que** ladite composition est telle que définie dans l'une quelconque des revendications 5 à 11.

## Patentansprüche

1. Zusammensetzung, enthaltend zumindest ein thermogelierendes Polymer in wässriger Lösung, wobei das thermogelierende Polymer einerseits wasserlösliche Einheiten und andererseits Einheiten enthält, die im Wasser eine untere kritische Entmischungstemperatur aufweisen, und aus Polyurethanen ausgewählt ist, die Gruppen von Poly(ethylenoxid-b-Propylenoxid-b-Ethylenoxid) (POE-b-POP-b-POE) enthalten, und in einer Massenkonzentration vorliegt, die geringer oder gleich 10 % in der wässrigen Lösung ist, und zwar für die Verwendung bei der Vorbeugung und/oder Behandlung von Schnarchen.

2. Zusammensetzung zur Verwendung nach Anspruch 1, **dadurch gekennzeichnet, dass** das thermogelierende Polymer in wässriger Lösung bei Raumtemperatur eine Viskosität von unter 0,1 Pa.s unter 10s⁻¹ Scherung und bei Körpertemperatur eine Viskosität von über 0,1 Pa.s, vorzugweise über 0,3 Pa.s, unter 10s⁻¹ Scherung bei Berührung der Schleimhäute aufweist.

3. Zusammensetzung zur Verwendung nach einem der Ansprüche 1 oder 2,
**dadurch gekennzeichnet, dass** das thermogelierende Polymer in einer Massenkonzentration von 2 bis 10 % in der wässrigen Lösung vorliegt.

4. Zusammensetzung zur Verwendung nach einem der Ansprüche 1 bis 3,
**dadurch gekennzeichnet, dass** das thermogelierende Polymer durch Polykondensation von Diisocyanaten und Triblockdiolen POE-b-POP-b-POE erhalten wird, die Gruppen von Harnstoff und/oder Allophanaten enthalten können.

5. Zusammensetzung zur Verwendung nach einem der Ansprüche 1 bis 4,
**dadurch gekennzeichnet, dass** sie einen Wirkstoff enthält, der in kontrollierter Weise freigesetzt wird.

6. Zusammensetzung zur Verwendung nach Anspruch 5, **dadurch gekennzeichnet, dass** der Wirkstoff mit dem Gel umhüllt ist, das bei Kontakt mit den Schleimhäuten entsteht, und in kontrollierter Weise freigesetzt wird.

7. Zusammensetzung zur Verwendung nach einem der Ansprüche 5 oder 6, **dadurch gekennzeichnet, dass** der Wirkstoff ausgewählt ist aus einem schwellungsmildernden Mittel, einem entzündungshemmenden Mittel, einem Mittel zur Behandlung bzw. Vorbeugung von Schnarchen und einem gefäßverengenden Mittel.

8. Zusammensetzung zur Verwendung nach einem der Ansprüche 1 bis 7, **dadurch gekennzeichnet, dass** sie zumindest ein Zusatzmittel enthält, ausgewählt aus:
- Elektrolyten,
- Schmiermitteln,
- Alkoholen,
- nichtionischen Tensiden, und
- Polysacchariden.

9. Zusammensetzung zur Verwendung nach einem der Ansprüche 1 bis 8,
**dadurch gekennzeichnet, dass** sie nasal oder oral verdampft werden kann.

10. Zusammensetzung zur Verwendung nach einem der Ansprüche 1 bis 9,
**dadurch gekennzeichnet, dass** sie an der Schleimhaut anhaftende Eigenschaften aufweist, die gestatten, dass sie gegen Auswaschung durch den Speichelfluss beständig ist.

11. Zusammensetzung zur Verwendung nach einem der Ansprüche 1 bis 9,
**dadurch gekennzeichnet, dass** sie die Schleimhäute schmierende Eigenschaften aufweist.

12. Verwendung von zumindest einem thermogelierenden Polymer, wie in einem der Ansprüche 1 bis 4 definiert, zur Herstellung einer Zusammensetzung zur Vorbeugung und/oder Behandlung von Schnarchen.

13. Verwendung nach Anspruch 12, **dadurch gekennzeichnet, dass** die Zusammensetzung derart ist, wie sie in einem der Ansprüche 5 bis 11 definiert ist.

## Claims

1. Composition comprising at least one thermogelling polymer in aqueous solution, said thermogelling polymer comprising on the one hand water-soluble units and on the other hand units having a lower critical solution temperature in water and being selected from polyurethanes having poly(ethylene oxide-b-propylene oxide-b-ethylene oxide) (PEO-b-PPO-b-PEO) groups and being present at a concentration by weight of less than or equal to 10% in said aqueous solution, for use in preventing and/or treating snoring.

2. Composition for use according to claim 1, **characterized in that** said thermogelling polymer displays, in aqueous solution, a viscosity below 0.1 Pa.s under 10s⁻¹ of shearing at room temperature and a viscosity above 0.1 Pa.s, preferably above 0.3 Pa.s, under 10s⁻¹ of shearing in contact with the mucosae, at body temperature.

3. Composition for use according to one of claims 1 or 2, **characterized in that** said thermogelling polymer is present at a concentration by weight from 2 to 10% in the aqueous solution.

4. Composition for use according to any one of claims 1 to 3, **characterized in that** said thermogelling polymer is obtained by polycondensation of diisocyanates and of triblock diols PEO-b-PPO-b-PEO and can comprise urea and/or allophanates groups.

5. Composition for use according to any one of claims 1 to 4, **characterized in that** it contains an active ingredient which is released in a controlled manner.

6. Composition for use according to claim 5, **characterized in that** the active ingredient is coated by the gel that has formed on contact with the mucosae and is released in a controlled manner.

7. Composition for use according to any one of claims 5 and 6, **characterized in that** said active ingredient is selected from a decongestant, an anti-inflammatory agent, an agent able of treating or preventing snoring and a vasoconstrictor agent.

8. Composition for use according to any one of claims 1 to 7, **characterized in that** it comprises at least one additive selected from
- electrolytes,
- lubricants,
- alcohols,
- non-ionic surfactants, and
- polysaccharides.

9. Composition for use according to any one of claims 1 to 8, **characterized in that** it is suitable for spraying, by the nasal or buccal route.

10. Composition for use according to any one of claims 1 to 9, **characterized in that** it has muco-adhesive properties, enabling it to resist being washed away by the flow of saliva.

11. Composition for use according to any one of claims 1 to 9, **characterized in that** it displays properties of lubrication of the mucosae.

12. Use of at least one thermogelling polymer as defined in any one of claims 1 to 4 for preparing a composition for preventing and/or treating snoring.

13. Use according to claim 12, **characterized in that** said composition is as defined in any one of claims 5 to 11.
